# EUROPÄISCHE PATENTANMELDUNG

(11) **EP 3 449 959 A1**
(43) Veröffentlichungstag der Anmeldung: **06.03.2019**
(21) Anmeldenummer: 18196369.5
(22) Anmeldetag: 27.07.2012
(51) Int. Cl.: A61M 1/16

(54) **ANORDNUNG ZUM ENTFERNEN VON KOHLENSTOFFDIOXID AUS EINEM EXKORPORALEN BLUTSTROM MITTELS INERTGASEN**

(30) Priorität: 27.07.2011 DE 102011052187
(62) Teilanmeldung aus: 12750340.7
(71) Anmelder: Joost, Thilo, 61169 Friedberg (DE)
(72) Erfinder: Joost, Thilo, 61169 Friedberg (DE); Köbrich, Rainer, 68809 Neulußheim (DE)
(74) Vertreter: Linhart, Friedrich Karl Eberhard

(57) **Zusammenfassung**

Die Erfindung betrifft eine Anordnung (10, 100, 200) zum Entfernen von Kohlenstoffdioxid aus einem exkorporalen Blutstrom. Die Anordnung (10, 100, 200) umfasst einen Filter (12), der eine Membran (16) hat, die einen Blutbereich (14) von einem Gasbereich (18) trennt. Der exkorporale Blutstrom ist durch den Blutbereich (14) des Filters (12) geführt. Ebenso ist durch den Gasbereich (18) ein Gasstrom eines Spülgases geführt, wobei das Spülgas ein Inertgas oder eine Mischung aus Inertgasen ist. Ferner betrifft die Erfindung ein Verfahren zum Entfernen von Kohlenstoffdioxid aus einem exkorporalen Blutstrom, bei dem ebenfalls als Spülgas ein Inertgas oder eine Mischung aus Inertgasen verwendet wird.

## Beschreibung

Die Erfindung betrifft eine Anordnung zum Entfernen von Kohlenstoffdioxid aus einem exkorporalen Blutstrom, die einen Filter umfasst, der eine Membran hat, die einen Blutbereich von einem Gasbereich trennt. Durch den Blutbereich des Filters ist der exkorporale Blutstrom und durch den Gasbereich des Filters eine Gasstroms eines Spülgases geführt.

In der Medizin werden bei schwer erkrankten Patienten sogenannte Oxygeneratoren verwendet, um aus dem Blut dieser Patienten Kohlenstoffdioxid zu entfernen und das Blut mit Sauerstoff anzureichern. Heutzutage werden fast ausschließlich Oxygeneratoren verwendet, die eine Membran umfassen, über die ein Blutbereich von einem Gasbereich getrennt ist. Das Blut wird dem Patienten aus einem Hauptgefäß entnommen und vorzugsweise mittels einer Blutpumpe in den Blutbereich des Oxygenerators befördert. Durch den Gasbereich wird zeitgleich ein Spülgas transportiert, wobei als Spülgas in der Regel entweder reiner Sauerstoff oder ein Gemisch aus Sauerstoff und Stickstoff verwendet wird. Insbesondere wird ein Gemisch aus 21 % Sauerstoff und 79 % Stickstoff, sogenannte AIR, verwendet. Das Spülgas wird insbesondere aus in medizinischen Einrichtungen vorhandenen Wandentnahmestellen entnommen und entspricht somit den Anforderungen medizinischer Gase.

Durch den Druckgradienten des Partialdrucks bzw. des Konzentrationsgradienten des Kohlenstoffdioxids wird Kohlenstoffdioxid von dem Blutbereich durch die Membran hindurch in den Gasbereich transportiert, wohingegen durch den Partialdruckgradienten des Partialdrucks des Sauerstoffes bzw. durch den Konzentrationsgradienten des Sauerstoffs dieser durch die Membran hindurch von dem Gasbereich in den Blutbereich transportiert wird, so dass das Blut mit Sauerstoff angereichert wird und gleichzeitig Kohlenstoffdioxid aus dem Blut entfernt wird. Die Menge an Kohlenstoffdioxid, die pro Zeiteinheit dem Blut entnommen wird, und die Menge an Sauerstoff, mit der das Blut pro Zeiteinheit angereichert wird, hängen zum einen von der Strömungsgeschwindigkeit des Spülgases durch den Gasbereich und zum anderen von der Fördergeschwindigkeit des Blutes durch den Blutbereich ab.

Bei bekannten Anordnungen umfasst das Spülgas immer Sauerstoff, so dass zwangsläufig eine Anreicherung des Blutes mit Sauerstoff erfolgt, auch wenn die Atemleistung des Patienten selbst ausreichen würde, um eine ausreichende Sauerstoffversorgung zu gewährleisten und es nur notwendig wäre, das Kohlenstoffdioxid aus dem Blut zu entfernen. Die unnötige Anreicherung des Blutes über den Oxygenerator kann eine hypoxisch pulmonale Vaso-Konstriktion mit einer entsprechenden Shuntverschiebung zur Folge haben, so dass der ohnehin schon erkrankte Patient weiter belastet wird und seine Genesung behindert wird. Abhängig von dem verwendeten Spülgas können darüber hinaus weitere Gase von dem Spülgas auf das Blut übertragen werden, was ebenfalls zu Irritationen des Patienten führen kann.

Es ist Aufgabe der Erfindung, eine Anordnung zum Entfernen von Kohlenstoffdioxid aus einem exkorporalen Blutstrom anzugeben, mit deren Hilfe das Kohlenstoffdioxid aus dem Blutstrom schonend entfernt werden kann.

Diese Aufgabe wird durch eine Anordnung mit den Merkmalen des Anspruchs 1 gelöst. Vorteilhafte Weiterbildungen der Erfindung sind in den abhängigen Ansprüchen angegeben.

Erfindungsgemäß wird die Aufgabe dadurch gelöst, dass als Spülgas ein Inertgas oder eine Mischung aus Inertgasen verwendet wird. Somit wird erreicht, dass das Spülgas keine Verbindung mit dem exkorporalen Blutstrom eingeht, so dass dem Blutstrom keine Gase zugeführt werden, sondern nur Kohlenstoffdioxid aus dem exkorporalen Blutstrom aufgrund des zwischen dem Blutbereich und dem Gasbereich bestehenden Partialdruckgradienten bzw. Konzentrationsgradienten entfernt wird.

Unter Inertgas wird in diesem Zusammenhang ein Gas verstanden, das keine Verbindung mit Blut eingeht.

Für den Fall, dass als Spülgas ein einziges Inertgas verwendet wird, wird vorzugsweise Stickstoff oder ein Edelgas, insbesondere Helium, Neon, Xenon, Argon oder Krypton, als Inertgas verwendet. Entsprechend wird bei der Verwendung einer Mischung von Intergasen als Spülgas eine Mischung aus Stickstoff und mindestens einem Edelgas oder eine Mischung aus mindestens zwei Edelgasen verwendet.

Als Filter wird insbesondere ein Oxygenerator verwendet, so dass auf einfache Weise schonend dem Blut Kohlenstoffdioxid entzogen werden kann. Es ist vorzugsweise eine Blutpumpe vorgesehen, mit deren Hilfe der Blutstrom durch den Blutbereich gefördert wird.

Ferner kann eine Gasfördereinheit zum Erzeugen des Gasstroms des Spülgases vorgesehen sein. Die Gasfördereinheit umfasst vorzugsweise einen Gasblender, über den das Spülgas aus mehreren Inertgasen gemischt werden kann. Des Weiteren kann die Gasfördereinheit einen Ventilator umfassen, über den die Gasströmung des Spülgases erzeugt wird. Alternativ kann auf eine Gasfördereinheit verzichtet werden. In diesem Fall wird die Strömung insbesondere durch den Druck des Inertgases bzw. der Inertgase, mit dem sie bereitgestellt werden, erzeugt.

Es ist vorteilhaft, wenn mindestens ein Vorratsbehälter vorgesehen ist, in dem das Spülgas aufgenommen ist. Der Vorratsbehälter ist über eine Zuführleitung mit dem Filter verbunden. Alternativ können auch mehrere Vorratsbehälter vorgesehen sein, wobei in diesem Fall eine Glasmischeinheit, ein sogenannter Gasblender, vorgesehen ist, über den die in den Vorratsbehältern aufgenommenen Gase zu dem Spülgas gemischt werden, bevor das Spülgas dann der Zuführleitung und über diese dem Filter zugeführt wird.

Bei einer besonders bevorzugten Ausführungsform ist eine Rückführleitung zum Rückführen des Spülgases von dem Filter in die Zuführleitung vorgesehen, so dass das Spülgas erneut verwendet werden kann. Somit können die Inertgase mehrfach verwendet werden, was insbesondere bei der Verwendung von Edelgasen als Inertgasen zu einer Kostenreduktion führt.

Im Bereich der Rückführleitung ist insbesondere eine Reinigungseinheit zum zumindest teilweisen Entfernen des Kohlenstoffdioxids aus dem durch die Rückführleitung geführten Gas vorgesehen. Die Reinigungseinheit entfernt insbesondere das gesamte in dem durch die Rückführleitung geführten Gas enthaltenen Kohlenstoffdioxid, so dass nach dem Durchlaufen der Reinigungseinheit das Gas kein Kohlenstoffdioxid mehr umfasst und somit erneut zum Durchströmen des Gasbereiches des Filters verwendet werden kann. Die Rückführleitung ist insbesondere ebenfalls an dem Gasblender angeschlossen, so dass über den Gasblender das geführte Gas mit dem dem Vorratsbehälter entnommen Gas gemischt werden kann und somit jederzeit die gewünschte Zusammensetzung des Spülgases erzeugt werden kann.

Zur Aufrechterhaltung einer einstellbaren Strömungsgeschwindigkeit in der Rückführleitung ist das System eine Gastransporteinheit zugeordnet (vorzugsweise eine Turbine) hinzugefügt. Die Transportleistung wird dem einstellbaren Gasfluß durch den Filter untergeordnet und kann von dem Anwender ausgewählt werden.

Ferner ist es vorteilhaft, wenn stromabwärts des Filters ein Sensor zur Ermittlung des Kohlenstoffdioxidanteils des Spülgases vorgesehen ist. Mit Hilfe dieses Sensors kann insbesondere überwacht werden, dass das dem Filter zugeführte Spülgas kein Kohlenstoffdioxid umfasst, so dass der gewünschte Partialdruckgradient bzw. Konzentrationsgradient des Kohlenstoffdioxids zwischen dem Gasbereich und dem Blutbereich hergestellt ist und die gewünschte Transferleistung von Kohlenstoffdioxid von dem Blutstrom in das Spülgas sichergestellt ist.

Zusätzlich oder alternativ kann auch ein Sensor zur Ermittlung des Kohlenstoffdioxidanteils des Spülgases stromabwärts des Filters vorgesehen sein, so dass über den Vergleich des Kohlenstoffdioxidanteils des Spülgases stromaufwärts und stromabwärts des Filters ermittelt werden kann, wie viel Kohlenstoffdioxid dem exkorporalen Blutstrom über den Filter entnommen wurde. Somit kann auf einfache Weise überwacht werden, ob die gewünschte voreingestellte Transferleistung des Kohlenstoffdioxids auch erreicht wurde. Insbesondere ist somit eine Überwachung des Zustands des Patienten möglich.

Ferner ist es vorteilhaft, wenn stromabwärts und/oder stromaufwärts des Filters jeweils ein Sensor zur Ermittlung der Strömungsgeschwindigkeit des Spülgases vorgesehen ist. Somit kann auf einfache Weise die Strömungsgeschwindigkeit des Spülgases überwacht werden und insbesondere über den Vergleich der ermittelten Strömungsgeschwindigkeit bzw. der ermittelten Strömungsgeschwindigkeiten mit einem voreingestellten Sollwert und eine entsprechende Regelung der Strömungsgeschwindigkeit sichergestellt werden, dass die voreingestellte Strömungsgeschwindigkeit auch tatsächlich eingehalten wird, so dass die gewünschte voreingestellte Transferleistung erreicht wird.

Ferner umfasst die Anordnung insbesondere eine Steuereinheit, die die Gasfördereinheit derart ansteuert, dass diese das Spülgas mit einer voreingestellten Strömungsgeschwindigkeit dem Filter zuführt. Die Gasfördereinheit ist dabei insbesondere derart dimensioniert, dass mit ihrer Hilfe Strömungsgeschwindigkeiten zwischen 0,1 l/min und 20 l/min realisiert werden können, so dass entsprechend große Bandbreite an Transferleistungen möglich ist und somit die zu entfernende Kohlenstoffdioxidmenge an den Zustand des Patienten möglichst genau angepasst werden kann.

Bei einer besonders bevorzugten Ausführungsform umfasst die Steuereinheit eine Eingabe- und/oder Ausgabeeinheit zur Ausgabe von Informationen an eine Bedienperson und/oder zur Eingabe von Informationen durch die Bedienperson. Die ausgegebenen Informationen umfassen insbesondere die mit Hilfe der Sensoren ermittelten Kohlenstoffdioxidanteile des Spülgases und/oder die ermittelten Strömungsgeschwindigkeiten, so dass die Bedienperson auf einfache Weise überwachen kann, ob dem Blutstrom die planmäßige Menge an Kohlenstoffdioxid entnommen wurde. Die eingegebenen Informationen umfassen vorzugsweise Steuerungsdaten, mit deren Hilfe die Bedienperson die Anordnung steuern kann. Insbesondere kann die Bedienperson über die Eingabeeinheit die gewünschte Strömungsgeschwindigkeit des Spülgases und/oder die Zusammensetzung des Spülgases einstellen, und somit auch sicherstellen, wie viel Kohlenstoffdioxid aus dem exkorporalen Blutstrom entfernt wird. Die Eingabe- und Ausgabeeinheit ist insbesondere als Touchscreen ausgebildet, so dass nur eine einzige Einheit zur Eingabe und Ausgabe notwendig ist und diese einzige Einheit einfach und intuitiv von der Bedienperson bedient werden kann. Somit werden Eingabefehler vermieden und eine einfache Handhabung erreicht.

Ein weiterer Aspekt der Erfindung betrifft ein Verfahren zum Entfernen von Kohlenstoffdioxid aus einem exkorporalen Blutstrom, bei dem ein Blutstrom durch einen durch eine Membran von einem Gasbereich getrennten Blutbereich eines Filters geführt wird und bei dem ein Gasstrom eines Spülgases durch den Gasbereich geleitet wird. Als Spülgas wird ein Inertgas oder eine Mischung aus Inertgasen verwendet, wobei als Inertgas insbesondere Stickstoff oder ein Edelgas bzw. als Mischung eine Mischung aus Stickstoff und mindestens einem Edelgas oder eine Mischung aus mindestens zwei Edelgase verwendet wird.

Weitere Merkmale und Vorteile der Erfindung ergeben sich aus der folgenden Beschreibung, die die Erfindung anhand von Ausführungsbeispielen in Zusammenhang mit den beigefügten Figuren näher erläutert.

Es zeigen:
- Figur 1: eine schematisch Darstellung einer Anordnung zum Entfernen von Kohlenstoffdioxid aus einem exkorporalen Blutstrom gemäß einer ersten Ausführungsform;
- Figur 2: eine schematische Darstellung einer Anordnung zum Entfernen von Kohlenstoffdioxid aus einem exkorporalen Blutstrom gemäß einer zweiten Ausführungsform; und
- Figur 3: eine schematische Darstellung einer Anordnung zum Entfernen von Kohlenstoffdioxid aus einem exkorporalen Blutstrom gemäß einer dritten Ausführungsform.

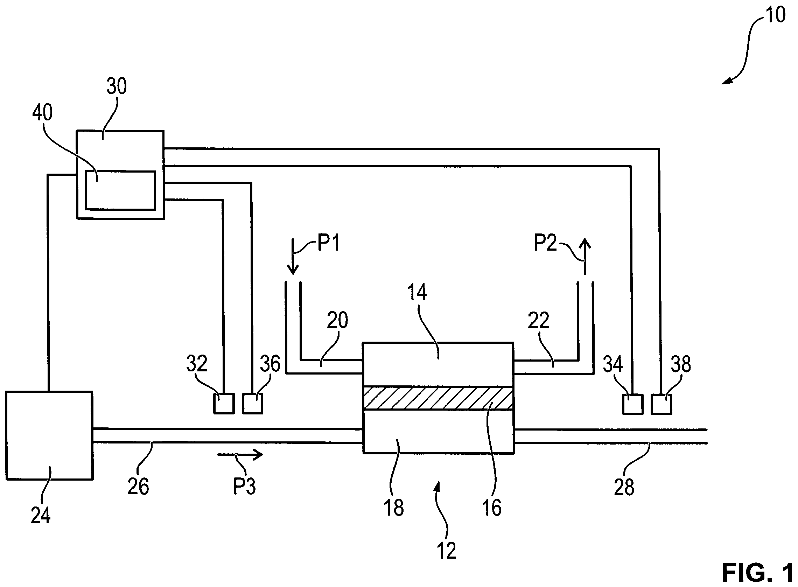

In Figur 1 ist eine schematische Darstellung einer Anordnung 10 zum Entfernen von Kohlenstoffdioxid aus einem exkorporalen Blutstrom eines Patienten dargestellt. Die Anordnung 10 umfasst einen als Oxygenerator 12 ausgebildeten Filter, der einen Blutbereich 14 und einen über eine Membran 16 von diesem Blutbereich 14 getrennten Gasbereich 18 aufweist. Durch den Blutbereich 14 wird der exkorporale Blutstrom entsprechend der Pfeile P1 und P2 durchgefördert, wozu eine Zuflussleitung 20 und eine Abflussleitung 22 vorgesehen sind.

Durch den Gasbereich 18 wird ein in einem Vorratsbehälter 24 aufgenommenes und über eine Zuführleitung 26 dem Gasbereich 18 zugeführte Spülgas gefördert, was durch den Pfeil P3 angedeutet ist.

Erfindungsgemäß wird als Spülgas ein Inertgas oder eine Mischung von Inertgasen verwendet, wobei als Inertgas jedes Gas verwendet werden kann, das keine Bindung mit Blut eingeht. Aufgrund der Druckdifferenz des Partialdrucks des Kohlenstoffdioxids zwischen dem Blutbereich 14 und dem Gasbereich 18 bzw. des Konzentrationsunterschieds zwischen dem Blutbereich 14 und dem Gasbereich 18 wird Kohlenstoffdioxid durch die Membran 16 hindurch dem Blutstrom entnommen und dem Spülgas zugeführt, so dass der Kohlenstoffdioxidgehalt des Blutstromes vermindert wird. Da das als Spülgas verwendete Inertgas bzw. die als Spülgas verwendete Mischung der Inertgase selbst keine Bindung mit dem Blut eingehen kann, erfolgt keine Anreicherung des Blutstroms mit dem Inertgas. Da das Inertgas insbesondere keinen Sauerstoff umfasst, erfolgt über den Oxygenerator 12 lediglich eine Entfernung des Kohlenstoffdioxids aus dem Blutstrom, aber keine Anreicherung des Blutstroms mit Sauerstoff. Somit wird der Patient, dem der Blutstrom entnommen ist, nicht durch das Einbringen von Sauerstoff irritiert, so dass insbesondere eine hypoxisch pulmonale Vaso-Konstriktion mit einer entsprechenden Shuntverschiebung vermieden wird. Hiermit werden auch weitere negative Einflüsse auf den blutgasbezogenen Versorgungszustand des bereits schon kritisch kranken Patienten vermieden. Unter einer Shuntverschiebung wird in diesem Zusammenhang verstanden, dass sich die Grenze, bis zu welcher die feinen Blutgefäße der Lunge mit durchblutet werden, verändert

Als Inertgas wird insbesondere Stickstoff oder ein Edelgas bzw. eine Mischung der zuvor genannten Gase verwendet. Als Edelgas werden insbesondere Helium, Neon, Argon, Krypton oder Xenon verwendet.

Das mit dem Kohlenstoffdioxid angereicherte Spülgas wird über die Abführleitung 28 abgeführt und beispielsweise einem Recyclingsystem zugeführt.

Ferner umfasst die Anordnung 10 eine Steuereinheit 30, zwei Kohlenstoffdioxidsensoren 32, 34 zum Ermitteln des Kohlenstoffdioxidanteils des Spülgases und zwei Strömungsgeschwindigkeitssensoren 36, 38 zum Ermitteln der Strömungsgeschwindigkeit des Spülgases. Jeweils ein Kohlenstoffdioxidsensor 32, 34 und ein Strömungsgeschwindigkeitssensor 36, 38 sind stromaufwärts des Oxygenerators 12 und stromabwärts des Oxygenerators 12 angeordnet.

Die Steuereinheit 30 weist einen Touchscreen 40 auf, mit dessen Hilfe Informationen an eine Bedienperson der Anordnung 10 ausgegeben werden können und Informationen durch die Bedienperson, insbesondere Daten zur Steuerung der Anordnung 10, wie zum Beispiel eine Sollmenge von aus dem exkorporalen Blutstrom zu entnehmende Kohlenstoffdioxidmenge, eingegeben werden können. Die mit Hilfe der Sensoren 32 bis 38 ermittelten Werte werden insbesondere über den Touchscreen 40 der Bedienperson angezeigt, so dass die Bedienperson auf einfache Weise die planmäßige Funktion der Anordnung 10 überwachen kann. Insbesondere ermittelt die Steuereinheit 30 einen Differenzwert aus den mit Hilfe des Kohlenstoffdioxidsensors 34 ermittelten Wertes und dem mit Hilfe des Kohlenstoffdioxidsensors 32 ermittelten Wertes, so dass über eine, insbesondere grafische, Anzeige dieses Differenzwertes die Bedienperson auf einfache Weise erkennen kann, wie viel Kohlenstoffdioxid dem Blutstrom entnommen wurde.

Ferner kann die Bedienperson über die Steuereinheit 30 die Strömungsgeschwindigkeit, mit der das Spülgas den Gasbereich 18 durchströmen soll, einstellen, so dass über die Strömungsgeschwindigkeit die Transferleistung des Kohlenstoffdioxids, d.h. der Anteil des Kohlenstoffdioxids der dem Blutstrom entnommen wird, auf einfache Weise gesteuert werden kann. Die Strömungsgeschwindigkeit ist insbesondere in einem Bereich zwischen 0,1 l/min und 20 l/min einstellbar. Hierzu steuert die Steuereinheit 30 den Vorratsbehälter 24, insbesondere ein Ventil des Vorratsbehälters 24, derart an, dass dieser einen Spülgasstrom mit entsprechender Strömungsgeschwindigkeit in die Zuführleitung 26 einleitet und somit dem Gasbereich 18 zuführt.

Bei einer bevorzugten Ausführungsform ist über den Strömungsgeschwindigkeitssensor 36 und/oder den Strömungsgeschwindigkeitssensor 38 ein Regelkreis ausgebildet. In diesem Fall vergleicht die Steuereinheit 30 den über den Strömungsgeschwindigkeitssensor 36 und/oder über den Strömungsgeschwindigkeitssensor 38 ermittelten Ist-Wert der Strömungsgeschwindigkeit des Spülgases mit dem voreingestellten Soll-Wert der Strömungsgeschwindigkeit und steuert den Vorratsbehälter 34 derart an, dass der Ist-Wert dem Soll-Wert entspricht.

Bei einer alternativen Ausführungsform kann die Strömung des Spülgases auch nicht ausschließlich über den Druck, mit dem das Spülgas in dem Vorratsbehälter 24 aufgenommen ist, erzeugt werden, sondern eine weitere separate Gasfördereinheit zum Erzeugen des Stroms des Spülgases durch den Gasbereich 18 vorgesehen sein.

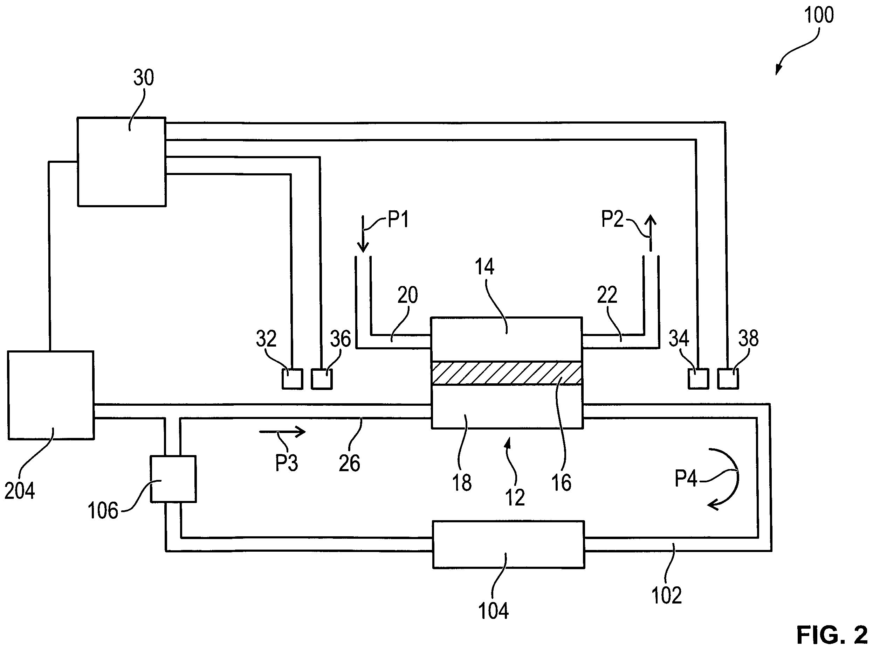

In Figur 2 ist eine schematische Darstellung einer Anordnung 100 zum Entfernen von Kohlenstoffdioxid aus einem exkorporalen Blutstrom gemäß einer zweiten Ausführungsform dargestellt. Die zweite Ausführungsform unterscheidet sich von der in Figur 1 gezeigten ersten Ausführungsform dadurch, dass das Spülgas nach dem Durchströmen des Gasbereiches 18 nicht entsorgt wird, sondern über eine Rückführleitung 102, wie durch den Pfeil P4 angedeutet, der Zuführleitung 26 zugeführt wird, so dass das Spülgas mehrfach verwendet werden kann. Somit ist es nicht notwendig, ständig "frisches" Spülgas aus dem Vorratsbehälter 24 zu entnehmen und dem Gasbereich 18 zuzuführen.

Im Bereich der Rückführleitung 102 ist eine Reinigungseinheit 104 zum Entfernen von Kohlenstoffdioxid aus dem durch die Rückführleitung zurückgeführten Spülgas vorgesehen. Diese Reinigungseinheit 104 ist insbesondere derart ausgebildet, dass mit ihrer Hilfe das Kohlenstoffdioxid vollständig aus dem Spülgas entfernt werden kann, so dass der Zuführleitung 26 nur reines Spülgas ohne Kohlenstoffdioxid zugeführt wird. Alternativ kann auch nur ein Teil des Kohlenstoffdioxids entfernt werden. Die Reinigungseinheit 104 ist insbesondere derart ausgebildet, dass das Spülgas in ihr durch chemische Prozesse wie unter Adsorption durch eine Behälter mit Soda oder Sodaline oder ausgestattet mit einer weiteren permeable Membran geleitet wird , durch das bzw. die das in dem Spülgas enthaltene Kohlenstoffdioxid gebunden und somit aus dem Spülgas entfernt wird, geleitet wird.

Ferner ist in der Rückführleitung 102 ein Ventilator 106 angeordnet, durch den die Gasströmung des Spülgases aufrechterhalten wird.

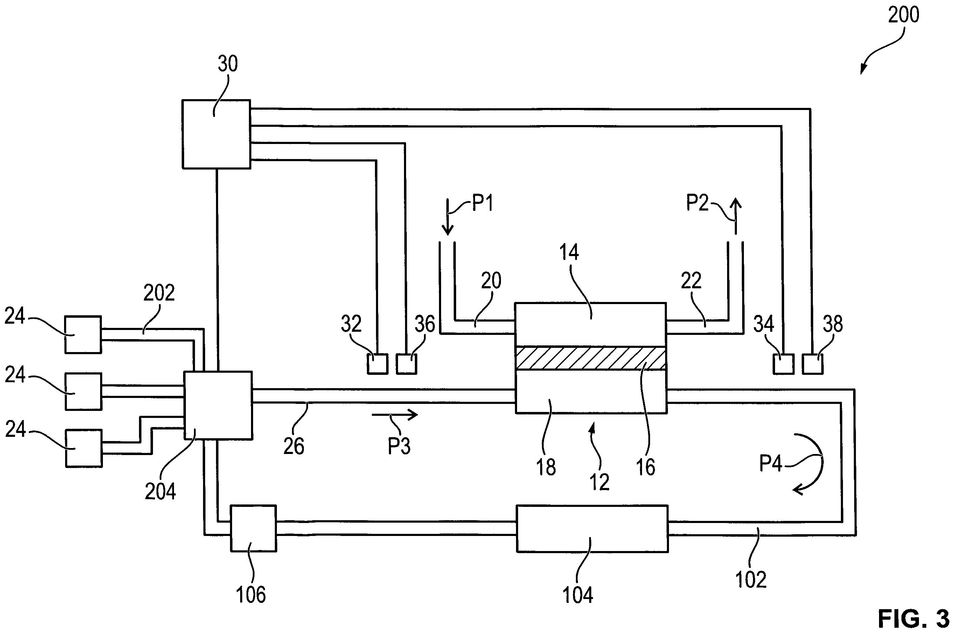

In Figur 3 ist eine schematische Darstellung einer Anordnung 200 zum Entfernen von Kohlenstoffdioxid aus einem exkorporalen Blutstrom gemäß einer dritten Ausführungsform dargestellt. Bei dieser dritten Ausführungsform weist die Anordnung 200 entgegen der ersten beiden Ausführungsformen nicht nur einen mit einem Gas befüllten Vorratsbehälter 24, sondern drei mit jeweils einem Gas befüllte Vorratsbehälter 24 auf. Die Vorratsbehälter 24 sind über jeweils eine Leitung 202 mit einer Gasmischeinheit 204, die auch als Gasblender bezeichnet wird, verbunden, so dass über die Gasmischeinheit 204 aus den in den Vorratsbehältern 24 aufgenommenen Gasen das Spülgas, das anschließend von dem Gasblender 204 zu dem Oxygenerator 12 gefördert wird, gemischt wird. In den Vorratsbehältern 24 ist insbesondere jeweils ein Inertgas aufgenommen, so dass über die Gasmischeinheit 204 ein Gemisch aus diesen Inertgasen als Spülgas gemischt werden kann. Alternativ kann auch in einem oder mehre Vorratsbehälter 24 bereits eine Mischung aus Inertgasen aufgenommen sein.

Über die Gasmischeinheit 204 kann auch lediglich eines der in den Vorratsbehältern 24 aufgenommenen Gase der Zuführleitung 26 zugeführt werden, so dass in diesem Fall lediglich dieses eine Gas als Spülgas dient. Somit kann die Gasmischeinheit 204 zum einen dazu dienen, aus mehreren Gasen ein Spülgas zu mischen, und zum anderen auch dazu, um eine Wahlmöglichkeit des Spülgases zu ermöglichen, ohne dass hierfür der Vorratsbehälter 24 gewechselt werden muss.

Die Rückführleitung 102 ist insbesondere auch an der Gasmischeinheit 204 angeschlossen, so dass das rückgeführte Spülgas über die Gasmischeinheit 204 mit eventuell den Vorratsbehältern 24 entnommenen Gasen gemischt werden kann. Alternativ kann die Verbindung der Rückführleitung 102 mit der Zuführleitung 26 auch stromabwärts der Gasmischeinheit 204 erfolgen.

Ferner ist alternativ möglich, dass mehr als drei Vorratsbehälter 24, beispielsweise vier Vorratsbehälter 24, oder auch nur zwei Vorratsbehälter 24 vorgesehen sind.

Des Weiteren ist es alternativ möglich, dass nur in einem Teil der Vorratsbehälter 24 Inertgase und in den anderen Vorratsbehältern 24 keine Inertgase, beispielsweise Sauerstoff oder ein Sauerstoff-Stickstoff-Gemisch, aufgenommen sind. Hierdurch wird erreicht, dass bei Patienten, bei denen nicht nur Kohlenstoffdioxid aus dem Blutstrom entfernt werden soll, sondern der Blutstrom auch mit Sauerstoff angereichert werden muss, beispielsweise weil die eigene Atemleistung des Patienten hierfür nicht ausreicht, dies über dieselbe Anordnung 200 erfolgen kann.

Die Zusammensetzung des Spülgases aus den in den Vorratsbehältern 24 aufgenommenen Gasen kann insbesondere über die Steuereinheit 30 eingestellt werden. Somit kann die Zusammensetzung des Spülgases einfach an die jeweiligen patientenabhängigen Gegebenheiten angepasst werden.

Bei einer weiteren alternativen Ausführungsform der Erfindung können auch wie bei der dritten Ausführungsform mehrere Vorratsbehälter 24 vorgesehen sein, aus deren Gasen mit Hilfe einer Gasmischeinheit 204 das Spülgas gemischt werden kann, und auf der anderen Seite aber, wie bei dem ersten Ausführungsbeispiel nach Figur 1, keine Rückführung des Spülgases nach dem Durchlaufen des Oxygenerators 12 erfolgen. In diesem Fall wird das Spülgas nach dem Durchlaufen des Oxygenerators 12 entsorgt und/oder recycelt.

### Bezugszeichenliste

- 10, 100, 200: Anordnung
- 12: Oxygenerator
- 14: Blutbereich
- 16: Membran
- 18: Gasbereich
- 20, 22: Leitung
- 24: Vorratsbehälter
- 26: Zuführleitung
- 28: Abführleitung
- 30: Steuereinheit
- 32, 34: Kohlenstoffdioxidsensor
- 36, 38: Strömungsgeschwindigkeitssensor
- 40: Touchscreen
- 102: Rückführleitung
- 104: Reinigungseinheit
- 106: Ventilator
- 202: Leitung
- 204: Gasmischeinheit
- P1, P2, P3, P4: Richtung

## Patentansprüche

1. Anordnung zum Entfernen von Kohlenstoffdioxid aus einem exkorporalen Blutstrom,
mit einem Filter (12), der eine Membran (16) umfasst, die einen Blutbereich (14), durch den der exkorporale Blutstrom geführt ist, von einem Gasbereich (18), durch den ein Gasstrom eines Spülgases geführt ist, trennt,
**dadurch gekennzeichnet, dass** das Spülgas ein Inertgas oder eine Mischung aus Inertgasen ist.

2. Anordnung (10, 100, 200) nach Anspruch 1, dadurch gezeichnet, dass das Inertgas Stickstoffstoff, Helium, Neon, Argon, Xenon oder Krypton ist.

3. Anordnung (10, 100, 200) nach Anspruch 1, **dadurch gekennzeichnet, dass** die Mischung eine Mischung aus Stickstoff, Helium, Neon, Argon, Xenon und/oder Krypton ist.

4. Anordnung (10, 100, 200) nach Anspruch 1, **dadurch gekennzeichnet, dass** die Anordnung (10, 100, 200) eine Gasfördereinheit zum Erzeugen des Gasstroms des Spülgases und/oder eine Blutpumpe zum Fördern des Blutstroms durch den Blutbereich (14) umfasst.

5. Anordnung (10, 100, 200) nach einem der vorhergehenden Ansprüche, **dadurch gekennzeichnet, dass** der Filter (12) einen Oxygenerator umfasst.

6. Anordnung (10, 100, 200) nach einem der vorhergehenden Ansprüche, **dadurch gekennzeichnet, dass** die Gasfördereinheit eine Gasmischeinheit (204) und/oder einen Ventilator (106) umfasst.

7. Anordnung (10, 100, 200) nach einem der vorhergehenden Ansprüche, **dadurch gekennzeichnet, dass** mindestens ein Spülgasvorratsbehälter (24) vorgesehen ist, in dem das Spülgas aufgenommen ist, und dass der Spülgasvorratsbehälter (24) über eine Zuführleitung (26) mit dem Filter (12) verbunden ist.

8. Anordnung (10, 100, 200) nach Anspruch 7, **dadurch gekennzeichnet, dass** eine Rückführleitung (102) zum Rückführen des Spülgases von dem Filter (12) in die Zuführleitung (26) vorgesehen ist.

9. Anordnung (10, 100, 200) nach Anspruch 8, **dadurch gekennzeichnet, dass** im Bereich der Rückführleitung (102) eine Reinigungseinheit (104) zum Entfernen von Kohlenstoffdioxid aus dem durch die Rückführleitung (102) geführten Gas angeordnet ist.

10. Anordnung (10, 100, 200) nach einem der vorhergehenden Ansprüche, **dadurch gekennzeichnet, dass** stromabwärts und/oder stromaufwärts des Filters (12) jeweils ein Sensor (32, 34) zur Ermittlung des Kohlenstoffdioxidanteils des Spülgases vorgesehen ist.

11. Anordnung (10, 100, 200) nach einem der vorhergehenden Ansprüche, **dadurch gekennzeichnet, dass** stromabwärts und/oder stromaufwärts Filters (12) jeweils ein Sensor (36, 38) zur Ermittlung der Strömungsgeschwindigkeit des Spülgases vorgesehen ist.

12. Anordnung (10, 100, 200) nach einem der vorhergehenden Ansprüche, **dadurch gekennzeichnet, dass** eine Steuereinheit (30) die den Gasstrom derart ansteuert, dass das Spülgas mit einer voreingestellten Strömungsgeschwindigkeit, insbesondere einer Strömungsgeschwindigkeit zwischen 0,1 l/min und 20 l/min, dem Filter (12) zuführt wird.

13. Anordnung (10, 100, 200) nach Anspruch 12, **dadurch gekennzeichnet, dass** die Steuereinheit (30) eine Eingabe- und/oder Ausgabeeinheit, insbesondere einen Touchscreen (40), zur Ausgabe von Informationen an eine Bedienperson und/oder zur Eingabe von Informationen, insbesondere zur Eingabe von Steuerungsdaten, durch eine Bedienperson umfasst.

14. Verfahren zum Entfernen von Kohlenstoffdioxid aus einem exkorporalen Blutstrom,
bei dem ein Blutstrom durch einen durch eine Membran (16) von einem Gasbereich (18) getrennten Blutbereich (14) eines Filters (12) geführt wird, und
bei dem ein Gasstrom eines Spülgases durch den Gasbereich (18) des Filters (12) geleitet wird,
**dadurch gekennzeichnet, dass** als Spülgas ein Inertgas oder eine Mischung aus Inertgasen verwendet wird.

15. Verfahren nach Anspruch 14, **dadurch gekennzeichnet, dass** als Inertgas Stickstoff, Helium, Neon, Argon, Xenon oder Krypton bzw. als Mischung aus Stickstoff, Helium, Neon, Argon, Xenon und/oder Krypton verwendet wird.
